# FASCICULE DE BREVET EUROPEEN

(11) **EP 2 126 142 B1**
(45) Date de publication et mention de la délivrance du brevet: **10.11.2010**
(21) Numéro de dépôt: 08761800.5
(22) Date de dépôt: 24.01.2008
(51) Int. Cl.: C13K 11/00, C12N 9/92, C12P 19/24, C13D 3/12, C13D 3/14, C13D 3/16, A23L 1/09

(54) **PROCEDE ET INSTALLATION DE PREPARATION DE SIROPS DE SUCRES DE FRUITS A HAUTE TENEUR EN FRUCTOSE**
VERFAHREN UND ANLAGE ZUR HERSTELLUNG VON FRUCHTZUCKERSIRUP MIT HOHEM FRUCTOSEGEHALT
METHOD AND EQUIPMENT FOR PRODUCING FRUIT SUGAR SYRUPS HAVING HIGH FRUCTOSE CONTENT

(30) Priorité: 01.02.2007 FR 0700712
(43) Date de publication de la demande: 02.12.2009
(73) Titulaire: Nutritis, 82000 Montauban (FR); Institut National Des Sciences Appliquees Toulouse, 31077 Toulouse Cedex 4 (FR)
(72) Inventeur: LAPOUJADE, Pierre, F-82100 Castelsarrasin (FR); GUIBERT, Alain, F-31750 Escalquens (FR); OUARNE, Françoise, F-31320 Castanet Tolosan (FR)
(74) Mandataire: Cabinet BARRE LAFORGUE & associés
(86) Numéro de dépôt international: PCT/FR2008/000085
(87) Numéro de publication internationale: WO 2008/107560

(56) Documents cités:
- WO-A1-03/016577
- ES-A1- 2 136 553
- GB-A- 949 293
- GB-A- 2 172 288
- SU-A1- 583 137
- US-A- 4 276 379
- US-A- 4 710 231

## Description

L'invention relève du domaine des sirops de sucres à teneur élevée en fructose. Elle concerne plus particulièrement un procédé et une installation de préparation de sirops de sucres à forte teneur en fructose, à partir de fruits.

Apprécié pour ses propriétés organoleptiques (flaveurs fruitées, sensation de fraîcheur en bouche) et ses vertus diététiques (pouvoir sucrant égal à 1,5 fois celui du saccharose, faible vitesse d'absorption intestinale, métabolisation indépendante de l'insuline, bas index glycémique), le fructose est aujourd'hui un produit d'intérêt industriel. Sa consommation n'est plus réduite à celle des fruits, elle est désormais largement associée à celle de nombreux produits transformés de l'industrie agroalimentaire (édulcorants, boissons sucrées, confitures, crèmes glacées, pâtisseries...).

A l'échelle industrielle, la préparation du fructose implique l'utilisation d'une enzyme à activité glucose-isomérase (FR 2 073 697) permettant la conversion du glucose en fructose. Dans ce contexte et jusqu'à présent, principalement deux types de matières premières végétales ont été proposés comme source de glucose :
- les plantes riches en amidon (essentiellement, le maïs, le blé, la pomme de terre...), à partir desquelles on extrait l'amidon que l'on hydrolyse en glucose ; ce glucose est finalement converti en fructose ;
- les plantes saccharifères (essentiellement la canne à sucre, les betteraves sucrières), à partir desquelles on extrait un jus que l'on traite (par exemple, selon la méthode décrite par US 6,406,548) pour obtenir un jus sucré clarifié et déminéralisé de saccharose ; ce saccharose est ensuite hydrolysé en une composition de sucres simples, de glucose et de fructose (par exemple, selon
la méthode décrite par US 6,916,381) ; le glucose est purifié puis converti en fructose et va enrichir la fraction de fructose initiale. GB 949 293 concerne procédé de préparation d'un sirop de sucres à haute teneur en fructose, dans lequel on hydrolyse le saccharose en fructose et glucose, en réalise une isomerisation en fructose du glucose et finalement on concentre.

Il a également été proposé de préparer des compositions concentrées en fructose par hydrolyse de l'inuline (un polysaccharide de fructose) recueilli des racines de la chicorée, des oignons du dahlia, des tubercules du topinambour.

L'invention a pour objectif de proposer un procédé de préparation de sirops de sucres à teneur élevée en fructose qui puisse être mis en oeuvre à partir de produits de départ autres que des plantes saccharifères, des plantes riches en amidon et/ou des plantes riches en inuline.

Dans tout le texte, l'expression « sirop de sucres à teneur élevée en fructose » est utilisée pour désigner une composition concentrée en sucres contenant au moins 95 % de fructose, par rapport au poids total de matière sèche, et/ou au moins 98 % de fructose, par rapport au poids total de sucres. A des fins de simplification, on pourra aussi utiliser l'expression « sirop de fructose selon l'invention » pour désigner un tel sirop de sucres.

L'invention vise à proposer un procédé destiné à être mis en oeuvre à l'échelle industrielle et qui, notamment par son fonctionnement, par le choix de la matière de départ et par la qualité du produit final, permet de concurrencer les procédés traditionnels de préparation de « sirops de fructose ».

L'invention vise en outre à proposer une installation industrielle permettant la mise en oeuvre d'un tel procédé.

A cet effet, l'invention concerne un procédé de préparation d'un sirop de sucres à haute teneur en fructose, dans lequel :
- à partir d'au moins une matière première végétale de départ, on prépare un jus sucré clarifié et déminéralisé,
- on traite ledit jus sucré clarifié et déminéralisé de façon à hydrolyser le saccharose en fructose et en glucose ; on obtient alors une composition de sucres simples comprenant une fraction de fructose, dite première fraction de fructose, et une fraction de glucose,
- on sépare la fraction de glucose de la première fraction de fructose, et on réalise une isomérisation en fructose du glucose contenu dans cette fraction de glucose, pour former une nouvelle fraction de fructose, dite seconde fraction de fructose,
- on combine les première et seconde fractions de fructose, et on les concentre en un sirop de sucres riche en fructose.

Un procédé de préparation selon l'invention est **caractérisé en ce que :**
1) au moins une matière première végétale de départ est issue d'au moins un fruit contenant naturellement du sorbitol,
2) il comprend une étape d'élimination au moins partielle du sorbitol.

Avantageusement et selon l'invention, ladite matière végétale est issue d'au moins un fruit choisi parmi : les pommes, les poires, les prunes, les pruneaux, les pêches, les nectarines, les abricots, les raisins. Autrement dit, un procédé de préparation d'un sirop de sucre à haute teneur en fructose selon l'invention est **caractérisé en ce qu**'il est mis en oeuvre à partir d'au moins un fruit choisi parmi : les pommes, les poires, les prunes, les pruneaux, les pêches, les nectarines, les abricots, les raisins.

Avantageusement et selon l'invention, à partir de ladite matière végétale, on extrait un premier jus que l'on traite pour obtenir un jus sucré clarifié et déminéralisé de couleur inférieure à 45 ICUMSA, et présentant un taux de cendres conductimétriques inférieur à 0,4 %. Ce premier jus peut être obtenu par toute technique d'extraction de jus de fruits -notamment par broyage-filtration et/ou par pressage-à partir d'un fruit ou de plusieurs fruits. Ce premier jus peut être également constitué d'un mélange de plusieurs jus de fruits obtenus chacun par extraction de jus de fruits à partir d'un ou plusieurs fruits.

Un procédé selon l'invention est aussi **caractérisé en ce qu'**il comprend également une étape d'élimination au moins partielle du sorbitol. Ce sorbitol est naturellement présent dans au moins un fruit entrant dans la composition de la matière première végétale de départ.

L'invention concerne ainsi un procédé de préparation de sirops de sucres à teneur élevée en fructose spécifiquement adapté à la transformation de fruits dans lesquelles du sorbitol est naturellement présent, tels que les pommes, les poires, les prunes, les pruneaux, les pêches, les nectarines, les abricots, les raisins. Outre de faire partie des fruits les plus produits en Europe, et en particulier en France, les fruits visés par l'invention connaissent chaque année un taux de retrait élevé. L'invention leur ouvre avantageusement une nouvelle voie de transformation industrielle.

Un procédé selon l'invention est du type comprenant une étape de séparation d'une fraction de glucose et d'une fraction de fructose après hydrolyse du saccharose, et une étape de conversion du glucose en fructose ; tel est le cas de la préparation des sirops de fructose jusqu'à présent mise en oeuvre à partir de plantes saccharifères.

A cet égard, il est à noter que les propriétés physicochimiques et la composition de fruits visés par l'invention (les pommes, les poires, les prunes, les pruneaux, les pêches, les nectarines, les abricots, les raisins), qui sont bien différentes de celles des plantes saccharifères, ne permettent pas d'appliquer à de telles matières premières en l'état les procédés d'extraction et de transformation tels qu'ils sont utilisés jusqu'à maintenant sur les plantes saccharifères, et rendent donc ces fruits *a priori* impropres pour une telle utilisation.

En particulier, dans un procédé selon l'invention, les paramètres de travail des étapes d'extraction du premier jus et de traitement de ce premier jus en un jus sucré décoloré et déminéralisé, sont précisément déterminés pour permettre de récupérer de façon simple et rapide les sucres des fruits : le fructose, mais aussi le saccharose et le glucose, qui sont également naturellement contenus dans ces fruits.

Egalement, dans un procédé selon l'invention, les étapes de clarification et de déminéralisation du premier jus (jus brut directement tiré de la matière de départ) sont exécutées selon des principes de purification et des paramètres de mise en oeuvre bien particuliers, spécifiquement déterminés pour conduire précisément à un jus sucré clarifié et déminéralisé de couleur inférieure à 45 ICUMSA, et présentant un taux de cendres conductimétriques inférieur à 0,4 %.

Les inventeurs ont en effet constaté que l'obtention d'un tel jus sucré décoloré et déminéralisé conditionne en grande partie le bon déroulement des étapes ultérieures, et en particulier :
- la préparation d'une composition de sucres simples, par hydrolyse du saccharose contenu dans le jus sucré décoloré et déminéralisé,
- la séparation du glucose et du fructose, et l'élimination du sorbitol,
- l'isomérisation du glucose en fructose,
et *in fine* l'obtention d'un sirop de fructose de qualité.

Avantageusement et selon l'invention, en vue d'obtenir un jus sucré clarifié et déminéralisé, on soumet le premier jus aux étapes de traitement suivantes :
- une centrifugation de l'ordre de 5 000-14 000 g,
- une ultrafiltration sur une membrane poreuse de seuil de coupure compris entre 1 kDa et 50 kDa,
- une électrodialyse ; les paramètres de travail sont choisis pour permettre une élimination au moins partielle des charges ioniques dudit premier jus ;
- une chromatographie sur une résine échangeuse d'anions et une chromatographie sur une résine échangeuse de cations.

Les étapes de préparation dudit jus sucré clarifié et déminéralisé sont exécutées dans cet ordre. Ceci n'exclut pas le fait qu'il puisse y avoir des interruptions (au cours de ces étapes de préparation et/ou entre deux étapes consécutives) et/ou des étapes supplémentaires venant s'intercaler entre ces différentes étapes.

Les paramètres de travail des étapes de centrifugation et d'ultrafiltration d'un procédé selon l'invention sont particulièrement choisis pour permettre une élimination au moins partielle de particules en suspension dans le premier jus et pour obtenir un jus clarifié de densité optique, mesurée à 650 nm, inférieure à 0,10 U. Pour ce faire, avantageusement et selon l'invention, la centrifugation est réalisée à 5 000 g, et l'ultrafiltration est réalisée avec une membrane d'ultrafiltration de seuil de coupure de l'ordre de 2,5 kDa et en appliquant une pression transmembranaire de l'ordre de 7 bars.

Selon un mode de réalisation particulier de l'invention, pour préparer le jus sucré décoloré et déminéralisé, avant de soumettre le premier jus à une centrifugation, on soumet celui-ci à l'action d'au moins une enzyme à activité pectolytique. A ce titre, on peut avantageusement utiliser les enzymes développées notamment pour l'industrie du vin et/ou des jus de fruits pour les étapes de débourbage et/ou de clarification.

Également, les paramètres de travail des étapes d'ultrafiltration et de chromatographie d'un procédé selon l'invention sont particulièrement choisis pour obtenir un jus déminéralisé ayant un taux de cendres conductimétriques inférieur à 0,4 %. A cet effet, avantageusement et selon l'invention, on réalise l'électrodialyse avec des paramètres de travail choisis pour permettre l'obtention d'une composition liquide de conductivité à 50°C inférieure à 800 µS.cm⁻¹. A titre d'exemple de mise en oeuvre, on utilise avantageusement des membranes cationiques CMXsb (MITSUBISHI) et des membranes anioniques AXE01 et/ou ASW (MITSUBISHI). La tension appliquée entre les membranes est de l'ordre de 14 V.

S'agissant des chromatographies sur résines échangeuses d'ions, on réalise avantageusement la chromatographie échangeuse de cations avec une résine cationique forte, et on réalise avantageusement la chromatographie échangeuse d'anions avec une résine anionique faible -éventuellement couplée à une résine anionique forte-.

De manière consacrée, les résines cationiques ou anioniques sont dites fortes ou faibles, selon leur aptitude à l'ionisation. Les résines cationiques fortes sont très fortement ionisées, quel que soit le pH ; il s'agit en particulier des résines à groupements sulfoniques. Les résines cationiques faibles, quant à elles, ne sont plus ionisées en milieu fortement acide ; il s'agit en particulier des résines à groupements carboxyliques et des résines à groupements carboxyméthyle. Les résines anioniques fortes les plus utilisées sont les résines à groupements aminés quaternaires et les résines à groupements aminés tertiaires. Les résines anioniques faibles les plus utilisées sont les résines à groupements aminés primaires et secondaires.

Selon l'invention, les chromatographies sur résines échangeuses d'ions sont réalisées en choisissant des paramètres de travail adaptés pour permettre l'obtention d'un jus sucré décoloré et déminéralisé ayant un taux de cendres conductimétriques inférieur à 0,4 %, de préférence inférieur à 0,2 %.

La teneur en cendres conductimétriques (qui est essentiellement liée à la teneur en sels minéraux, en matières inorganiques et en acides organiques) est traditionnellement déterminée par conductimétrie (c'est-à-dire par mesure de la conductivité électrique) sur une solution de sucre(s) de 28°B, à 20°C. Une conductivité de 3,13 µS/cm correspond à 0,0018 % de cendres conductimétriques.

Selon l'invention, le jus sucré décoloré et déminéralisé obtenu doit être ensuite soumis à une hydrolyse du saccharose. Pour ce faire, on réalise une hydrolyse enzymatique par une enzyme à activité β-D-fructofuranosidase (communément dénommée invertase). Avantageusement et selon l'invention, on utilise cette enzyme sous forme immobilisée.

Une fois l'hydrolyse du saccharose achevée, on récupère une composition de sucres simples principalement composée de glucose et de fructose, le glucose étant destiné à être converti en fructose. Pour convertir le glucose en fructose, on utilise avantageusement une enzyme à activité glucose-isomérase sous forme immobilisée.

Néanmoins, une composition de sucres simples préparée conformément à l'invention contient aussi du sorbitol, en quantité non négligeable. Ce sorbitol, qui provient d'au moins un fruit de la matière première végétale de départ et qui se retrouve aussi bien dans un jus sucré décoloré et déminéralisé que dans une composition de sucres simples préparée conformément à l'invention, doit être éliminé au moins partiellement au cours de la mise en oeuvre du procédé selon l'invention.

Dans ce contexte, les inventeurs ont déterminé que cette élimination au moins partielle du sorbitol pouvait être réalisée par chromatographie d'élution. Qui plus est, cette élimination pouvait être effectuée avec rapidité et simplicité par une chromatographie d'élution sélective entre le fructose et le sorbitol. Pour ce faire et selon un mode de mise en oeuvre préféré d'un procédé selon l'invention, on traite la composition de sucres simples par des étapes de purification et d'isomérisation comme décrites ci-après.

On soumet la composition de sucres simples à une chromatographie d'élution sur une colonne de résine cationique Ca²⁺ adaptée à la séparation glucose-fructose. On obtient alors une fraction de fructose et une fraction de glucose. Le sorbitol se trouve réparti dans ces deux fractions. Cette fraction de fructose correspond à la première fraction de fructose au sens de l'invention.

On soumet ensuite la fraction de glucose ainsi obtenue à une isomérisation du glucose en fructose. Puis, on réalise une chromatographie d'élution sur une colonne de résine cationique Ca²⁺ adaptée à la séparation glucose-fructose. On récupère une nouvelle fraction de fructose, qui correspond à la seconde fraction de fructose au sens de l'invention.

On combine lesdites première et seconde fractions de fructose pour former une nouvelle fraction de fructose. On soumet cette nouvelle fraction de fructose à une chromatographie d'élution sur une colonne de résine cationique Ca²⁺ adaptée à la séparation fructose-sorbitol. On choisit des paramètres de travail adaptés pour permettre d'obtenir une fraction de fructose finale de teneur en sorbitol inférieure ou égale à 5 %, par rapport au poids total de matière sèche (de la fraction).

Selon ce même mode de réalisation préféré de l'invention, avantageusement, on réalise les chromatographies d'élution au moyen d'une colonne de résine cationique Ca²⁺ comprenant deux vannes de sortie à fonctionnement alterné, adaptée pour pouvoir réaliser sur commande soit une séparation glucose-fructose soit une séparation fructose-sorbitol.

Avantageusement et selon l'invention, pour la réalisation d'une colonne de chromatographie d'élution selon l'invention, on utilise une résine AMBERLITE CR 1320 Ca²⁺ (société ROHM ET HASS, France).

Selon l'invention, pour obtenir un sirop de sucres à teneur élevée en fructose conforme à l'invention, on soumet ladite fraction de fructose finale à un traitement de finition en vue de sa déminéralisation, sa désodorisation, sa décoloration et l'élimination de la patuline potentiellement présente, et pour améliorer sa stabilité dans le temps. Pour ce faire, avantageusement et selon l'invention, ladite fraction de fructose finale est soumise à :
- une déminéralisation par chromatographie sur résines échangeuses d'ions,
- un traitement sur des charbons actifs,
- une étape de concentration.

Avantageusement et selon l'invention, ladite déminéralisation est effectuée sur un lit mélangé de deux résines : un échangeur cationique fortement acide et un échangeur anionique fortement basique. On choisit des paramètres de travail adaptés pour permettre l'obtention d'une composition au taux de cendres conductimétriques inférieur à 0,2 % -de préférence inférieur à 0,1 %-.

Avantageusement et selon l'invention, le traitement sur des charbons actifs est effectué à une température de l'ordre de 60°C. Ce traitement sur des charbons actifs permet une élimination de la patuline (une mycotoxine) potentiellement présente, ainsi qu'une élimination des couleurs résiduelles et des odeurs d'amine ; ces odeurs proviennent de l'utilisation des résines de chromatographie. Avantageusement et selon l'invention, on réalise une filtration à la sortie de ce traitement sur des charbons actifs pour piéger les particules de charbon détachées de la colonne. Pour ce faire, on utilise avantageusement un filtre stérilisant.

Avantageusement et selon l'invention, la concentration de la fraction de fructose finale est effectuée selon une technique d'évaporation sous vide à basse température, jusqu'à obtenir un sirop de sucres à teneur élevée en fructose, de concentration en sucres au moins de l'ordre de 70 %, par rapport au poids total de la composition (composition humide).

Selon un mode de mise en oeuvre préféré d'un procédé selon l'invention, pour prévenir les risques de contamination pouvant survenir au cours de la préparation du sirop de fructose, on concentre jusqu'à obtenir une concentration en sucres au moins de l'ordre de 60 %, par rapport à poids total de la composition, au moins une composition de sucres choisie parmi : ledit jus sucré décoloré et déminéralisé, ladite composition de sucres simples, une desdites fractions de fructose, ladite fraction de glucose. Avantageusement et selon l'invention, on procède par évaporation sous vide à basse température.

Le procédé selon l'invention permet d'obtenir un sirop de sucres à teneur élevée en fructose, dit sirop de fructose selon l'invention. En l'occurrence, un sirop de fructose selon l'invention se distingue des sirops de fructose préparés selon les méthodes antérieures d'extraction à partir de matière première végétale autre que des fruits, par la présence de sorbitol, au moins sous forme de traces. Cette présence de sorbitol atteste de l'utilisation d'un fruit comme matière première pour la préparation du sirop et permet l'identification d'un sirop de fructose selon l'invention. Cette identification peut par exemple être effectuée par une analyse en HPLC.

En particulier, un sirop de fructose selon l'invention présente une teneur en fructose au moins égale à 95 %, par rapport au poids total de matière sèche, et/ou au moins égale à 98 %, par rapport au poids total de sucres. Egalement, un sirop de fructose selon l'invention présente une teneur en sorbitol égale ou inférieure à 5 %, par rapport au poids total de matière sèche.

Avantageusement et selon l'invention, un sirop de fructose selon l'invention présente une concentration en sucres au moins de l'ordre de 70 %, par rapport au poids total de la composition.

L'invention concerne également une installation permettant la mise en oeuvre d'un procédé de préparation d'un sirop de fructose conforme à l'invention. Selon un mode de réalisation préféré d'une installation selon l'invention, celle-ci comprend :
- des moyens d'extraction adaptés pour permettre d'extraire un premier jus, à partir d'au moins une matière première végétale de départ issue d'au moins un fruit contenant naturellement du sorbitol -notamment choisi parmi les pommes, les poires, les prunes, les pruneaux, les pêches, les nectarines, les abricots, les raisins- ;
- un équipement de raffinage pour préparer, à partir dudit premier jus, un jus sucré décoloré et déminéralisé de couleur inférieure à 45 ICUMSA, et ayant un taux de cendres conductimétriques inférieur à 0,4 % -de préférence inférieur à 0,2 %-,
- un réacteur comprenant une enzyme à activité β-D-fructofuranosidase, avantageusement immobilisée, apte à permettre une hydrolyse du saccharose contenu dans le jus sucré décoloré et déminéralisé, et à permettre l'obtention d'une composition de sucres simples,
- des moyens permettant de réaliser une séparation d'une fraction de fructose et d'une fraction de glucose à partir de la composition de sucres simples,
- un réacteur comprenant une enzyme à activité glucose-isomérase, avantageusement immobilisée, apte à permettre une conversion du glucose en fructose,
- des moyens permettant une élimination du sorbitol.

Avantageusement et selon l'invention, ledit équipement de raffinage comprend :
- un dispositif de centrifugation,
- une colonne d'ultrafiltration équipée d'une membrane poreuse de seuil de coupure compris entre 1 kDa et 50 kDa -par exemple 2,5 kDa-,
- un électrodialyseur apte à fonctionner avec des paramètres de travail adaptés pour permettre l'obtention d'une composition liquide de conductivité à 50°C inférieure à 800 µS.cm⁻¹,
- une colonne de chromatographie échangeuse d'anions (comprenant avantageusement une résine anionique faible) et une colonne de chromatographie échangeuse de cations (comprenant avantageusement une résine cationique forte).

Avantageusement et selon l'invention, ladite installation comprend une colonne de chromatographie d'élution renfermant une résine cationique Ca²⁺ (par exemple, une résine AMBERLITE CR 1320 Ca²⁺ de la société ROHM ET HASS, France) et dotée de deux vannes de sortie à fonctionnement alterné, adaptée pour pouvoir réaliser sur commande soit une séparation glucose-fructose soit une séparation fructose-sorbitol.

Avantageusement et selon l'invention, ladite installation comprend un équipement adapté pour réaliser un traitement de finition d'une composition de fructose consistant en une déminéralisation, une désodorisation, une décoloration, une élimination de la patuline potentiellement présente, et une concentration. A cet effet, ledit équipement comprend :
- une colonne de chromatographie renfermant un lit mélangé de deux résines : un échangeur cationique fortement acide et un échangeur anionique fortement basique,
- une colonne de charbons actifs, couplée en sortie à un dispositif de filtration,
- un évaporateur fonctionnant sous vide et à basse température.

L'invention concerne également un procédé et une installation permettant la préparation d'un sirop de fructose selon l'invention, caractérisés en combinaison par tout ou partie des caractéristiques mentionnées ci-dessus ou ci-après.

D'autres buts, caractéristiques et avantages de l'invention apparaîtront à la lecture des exemples qui suivent et de la description qui se réfère aux figures annexées, donnés à titre non limitatif. Dans les figures :

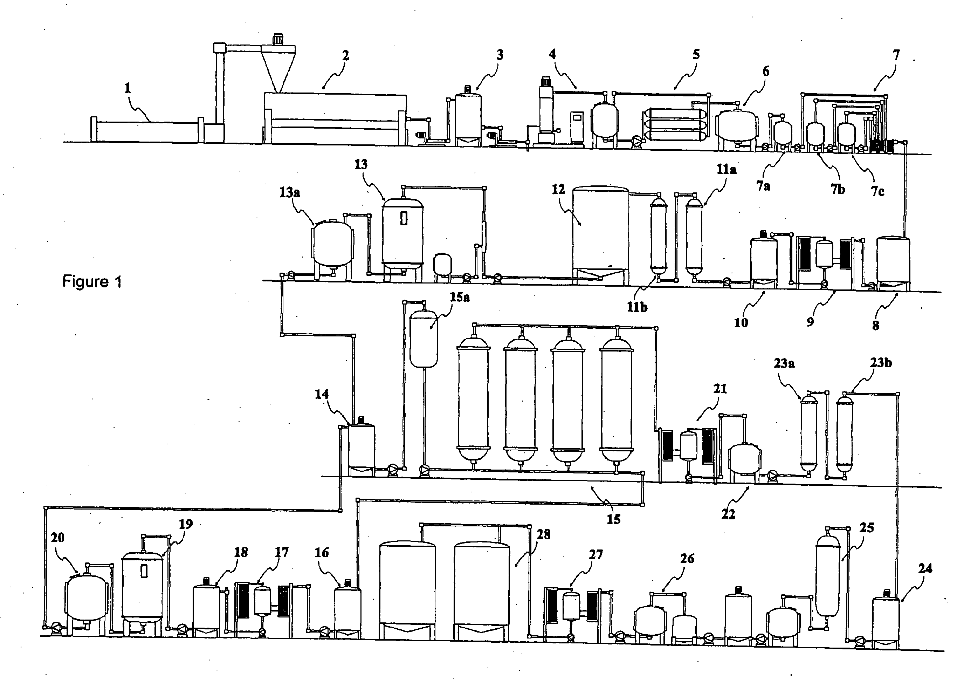

- la figure 1 illustre de façon schématique un exemple particulier d'installation permettant la préparation d'un sirop de fructose selon un mode de mise en oeuvre préféré, mais non limitatif, d'un procédé conforme à l'invention,
- la figure 2 correspond à un spectre HPLC d'un sirop de sucres à teneur élevée en fructose obtenu à partir de pommes, soumis à un procédé de transformation conforme à l'invention.

Le descriptif ci-après décrit concerne un mode de mise en oeuvre particulièrement préféré d'un procédé conforme à l'invention.

Les fruits, chargés sur un dispositif de convoyage hydraulique 1, sont lavés et transportés à l'intérieur d'un dispositif d'extraction par broyage-filtration 2. En sortie, un premier jus est obtenu.

Ledit premier jus est recueilli dans une première cuve de stockage 3, avant d'être transféré à l'intérieur d'un dispositif de centrifugation 4 en vue d'obtenir un jus clarifié dont la densité optique (DO), mesurée à 650 nm, est inférieure à 0,1 U.

Une installation pilote a été réalisée avec une centrifugeuse continue à bol vertical (tubulaire) de 6,3 litres de volume (modèle : SHARPLES type AS 16) fonctionnant avec les paramètres suivants :
- une accélération de 5 000 g,
- un temps de séjour de 10 minutes, pour un volume à traiter de l'ordre de 6 m³ par heure,
- une température de travail de l'ordre de 20°C.

Dans ces conditions, des abattements de DO de plus de 90 % ont pu être obtenus (valeurs obtenues pour un essai réalisé sur jus de pomme) :
- 94 % pour une suspension de DO initiale de 1,5 U (DO finale de 0,09 U),
- 91 % pour une suspension de DO initiale de à 1 U (DO finale de 0,09 U),
- 96 % pour une suspension de DO initiale de 1,5 U (DO finale de 0,06 U).

Dans la cuve 3, préalablement à l'étape de centrifugation, on peut envisager d'effectuer une préclarification par adjonction d'une enzyme à activité pectolytique. Ce faisant, après centrifugation dans les conditions opératoires précédemment définies, on obtient un surnageant de DO avoisinant 0,05 U, mesurée à 650 nm..

Le jus de fruit centrifugé et clarifié traverse alors une série de colonnes d'ultrafiltration 5, équipées de membranes d'ultrafiltration en polyéthersulfone de seuil de coupure 2,5 kDa.

A la sortie de cette étape d'ultrafiltration, on récupère dans une cuve 6 un filtrat décoloré.

Lorsque le débit d'ultrafiltration chute, le rétentat est lavé afin de minimiser les pertes en sucres.

Dans l'installation pilote, l'ultrafiltration est réalisée au moyen d'une colonne d'ultrafiltration de la société TIA (France), équipée de membranes d'ultrafiltration Thin-Film ayant une surface de 1,77 m².

Les pressions de travail applicables à ces membranes varient de 4,6 à 26 bars pour une température maximale de 50°C.

Chaque essai avec ce pilote a permis de traiter 100 litres de jus de pomme. Un taux de décoloration moyen de 90 % a été obtenu en procédant avec les paramètres de travail suivants :
- pompe de recirculation : débit de 900 l/h,
- PTM (pression transmembranaire) : 7 bars,
- débit de filtrat : 5,6 l/h par m² de surface de membrane (ce débit diminue régulièrement au cours de la manipulation ; une diminution de débit d'environ 3,5 % du débit initial tous les 10 litres).

Le jus de fruit passe ensuite à travers un électrodialyseur 7 afin de diminuer la concentration des sels et des acides organiques initialement présents dans le jus de fruit.

L'électrodialyse est une méthode de séparation qui s'applique à des solutions ioniques. Elle utilise d'une part un champ électrique qui met en jeu une force motrice du transport des ions en solution et, d'autre part, des membranes perméables aux ions, qui assurent la sélectivité du transport ionique et permettent d'extraire une part de la charge ionique des solutions.

Dans l'installation pilote, l'électrodialyseur AQUALYSEUR P1, commercialisé par la société EIVS (France), a été utilisé et testé avec deux jeux de membranes :
- un empilement de vingt cellules d'électrodialyse, chacune étant constituée d'une membrane cationique, de deux cadres séparateurs et d'une membrane anionique ; l'ensemble des cellules présente une surface effective totale de 0,138 m², soit 0,276 m² de membrane (membranes SELEMION AMV et CMV, de la compagnie ASAHI GLASS CORPORATION, Japon),
- un empilement EURB-10 fourni par la société EURODIA (France) ; les dix cellules actives sont composées de vingt-deux membranes cationiques CMX sb et de dix membranes anioniques AXE 01 (NEOSEPTA-TOKUYAMA CORPORATION, Japon) ; la surface active totale de cet empilement est de 0,2 m² ; concernant les électrodes, l'anode est réalisée en TiPt, et la cathode en inox.

L'électrodialyseur est formé de :
- trois cuves : la première 7a contient le jus à dessaler, la seconde 7b l'électrolyte, et la troisième 7c la saumure récupérée du dessalage ;
- trois pompes (avec des têtes à entraînement magnétique en polypropylène) qui autorisent des débits de recirculation jusqu'à 400 l.h⁻¹ ;
- des vannes à boules permettant de récupérer les produits traités et le réglage de leur débit.

Par cette étape, on réalise une prédéminéralisation qui permet d'extraire partiellement les acides organiques et leurs cations associés. L'élimination des acides organiques et cations associés entraîne une diminution de la conductivité du milieu. Cette diminution de conductivité a été suivie au cours du temps. Le taux de dessalage varie entre 50 et 97 % en fonction de la charge initiale en sels et du temps de traitement. Ce temps de traitement est par exemple de l'ordre d'une dizaine de minutes dans le cas de jus de pomme.

La concentration en sucres des solutions à déminéraliser par cette électrodialyse peut varier de 12 à 50 %, par rapport au poids total des solutions.

En sortie de l'électrodialyseur 7, le jus sucré présente une concentration en sucres généralement comprise entre 12 et 50 %, par rapport au poids total du jus, et une température de l'ordre de 40°C. Pour éviter les risques de contaminations des sirops dilués, une préconcentration est prévue à ce stade. Elle est réalisée sous vide à basse température jusqu'à une concentration en sucres de l'ordre de 60 %, par rapport au poids total de la composition.

A cet effet, le jus sucré sortant de l'électrodialyseur 7 est recueilli dans une cuve 8 puis est envoyé, vers un évaporateur sous vide 9. Le produit concentré est envoyé dans la cuve de stockage 10.

La décoloration et la déminéralisation sont finalisées au moyen d'une chromatographie échangeuse d'ions, réalisée sur deux lits de résines séparés. Pour ce faire, le jus prédécoloré et prédéminéralisé passe dans une première colonne 11a de résine cationique forte, puis dans une seconde colonne 11b de résine anionique faible.

Des pilotes ont été réalisés avec des colonnes en verre à double enveloppe, de la société NORMARVER (France), alimentées grâce à des pompes péristaltiques de type Masterflex^{®}. La première colonne contient 80 ml d'Amberlite^{™} FPC22H, une résine cationique forte. La deuxième colonne contient 80 ml d'Amberlite^{™} FPA51, une résine anionique faible.

Avant la première utilisation, ces résines ont été passivées par trois saturations au MgSO₄, puis régénérées.

Pour faciliter l'écoulement en tête de colonne, une couche de résine inerte (Amberlite^{™} RF14) a été rajoutée au-dessus des résines échangeuses d'ions.

La concentration en sucres des solutions à déminéraliser peut varier de 12 à 50 %, par rapport au poids total de la composition.

La durée de la phase de production dans un cycle est fonction du taux de prédéminéralisation. Le débit d'alimentation appliqué est de 5,5 BV.h⁻¹ (volume de produit passé dans la colonne par volume de lit de substrat, par heure) ; soit un débit de 440 ml.h⁻¹.

La phase de décoloration-déminéralisation peut être considérée comme achevée lorsque le taux de cendres conductimétriques en sortie de colonne anionique est inférieur à 0,4 %, de préférence inférieur à 0,1 %.

La résine cationique et la résine anionique sont régénérées, respectivement, avec des quantités d'HCl et de NaOH purs de 0,1 kg par litre de résine. La quantité d'eau nécessaire par régénération est de 20 litres pour un pilote de 2 litres.

Le jus sucré décoloré et déminéralisé obtenu à l'issue de cette première phase est récupéré dans une cuve 12 avant d'être soumis à l'hydrolyse du saccharose. Ce jus sucré concentré renferme principalement un mélange de saccharose, de fructose et de glucose, ainsi que du sorbitol.

L'hydrolyse du saccharose est réalisée dans le réacteur continu 13 à lit fixe, dans lequel se trouve immobilisée la β-D fructofuranosidase. Le réacteur 13 est alimenté en flux descendant.

Avant d'être envoyé dans le réacteur 13, le jus sucré décoloré et déminéralisé passe dans une cuve tampon, à l'intérieur de laquelle son pH est ajusté au pH optimum de catalyse de l'enzyme utilisée, en l'occurrence un pH de 4,5 pour une température de l'ordre de 30°C.

Le débit d'alimentation initial du réacteur 13 peut varier de 0,3 à 1 BV.h⁻¹ en fonction de la teneur initiale de saccharose du jus sucré décoloré et déminéralisé. En cours de réaction, la perte d'activité de l'enzyme est compensée par une augmentation de la température de façon à maintenir un taux d'hydrolyse du saccharose supérieur ou égal à 99 %. Le pas de variation de la température est de 1°C, jusqu'à 60°C. La composition de sucres simples obtenue à ce stade est désignée « inverti de fruit » dans les exemples ci-après.

La composition de sucres simples produite par cette hydrolyse contient essentiellement du glucose et du fructose (éventuellement des traces de saccharose) et une quantité non négligeable de sorbitol (très variable d'un fruit à l'autre, et d'une variété de fruit à une autre). Elle est acheminée dans une cuve 13 a.

Selon la variété de fruit, on peut obtenir des compositions de sucres simples avec des teneurs en sorbitol bien au-delà de 6 %, par rapport au poids total de matière sèche.

La composition de sucres simples est recueillie dans une cuve 14, puis envoyée à travers un ensemble de colonnes de chromatographie d'élution 15 réglé de façon à permettre une séparation glucose-fructose. La résine utilisée est une résine cationique Ca²⁺. La séparation est effectuée par utilisation d'eau osmosée. Afin d'éviter l'oxydation des résines, l'eau d'élution et la solution d'alimentation doivent être dégazées dans un ballon flash 15a.

A la sortie des colonnes de chromatographie, nous obtenons une fraction de fructose et une fraction riche en glucose. Le sorbitol précédemment présent dans la composition de sucres simples est réparti dans les deux fractions de fructose et de glucose.

S'agissant de la fraction de glucose sortant des colonnes 15 de chromatographie (issue de la chromatographie de séparation fructose-glucose), elle est recueillie dans un premier temps dans une cuve 16. Pour limiter le risque de contaminations, cette fraction est rapidement concentrée à une concentration en sucres de l'ordre de 60 %, par rapport au poids total de la composition, au moyen d'un évaporateur sous vide 17, fonctionnant à basse température. La fraction de glucose concentrée est récupérée dans une cuve 18.

L'étape d'isomérisation du glucose en fructose est réalisée en continu dans un réacteur à lit fixe 19, contenant une glucose-isomérase sous forme immobilisée. Le réacteur 19 est alimenté en flux descendant.

Avant isomérisation, la fraction de glucose concentrée passe dans une cuve tampon (non représentée sur la figure), où son pH est ajusté à 7, au moyen d'une solution de (K⁺, OH⁻).

La fraction de glucose concentrée, tamponnée et additionnée d'une solution de MgCl₂ (pour une concentration finale de 3mM) est envoyée dans le réacteur 19, avec un débit constant. En sortie de réacteur 19, dans une cuve 20, on récupère un sirop contenant de l'ordre de 48 % de fructose et 52 % de glucose, par rapport au poids total de matière sèche.

La perte d'activité de l'enzyme est compensée par une augmentation progressive de la température du réacteur 19. Le pas de variation de la température dans le réacteur 19 est de 1°C. La plage de températures du réacteur 19 est fixée entre 35°C et 60°C.

Pour séparer le glucose et le fructose du sirop recueilli dans la cuve 20, ce sirop est renvoyé vers les colonnes de chromatographie 15, via la cuve 14 à l'intérieur de laquelle il se mélange à la composition de sucres simples issue de l'étape d'hydrolyse du saccharose du jus sucré décoloré et déminéralisé.

S'agissant des fractions de fructose qui sortent des colonnes 15 de chromatographie, celles-ci sont recirculées dans ces mêmes colonnes 15 de chromatographie. Lors de ce nouveau passage, les colonnes de chromatographie sont réglées pour pouvoir réaliser une séparation fructose-sorbitol.

En particulier, les paramètres de fonctionnement des colonnes 15 sont adaptés pour permettre l'obtention d'une fraction de fructose finale de teneur en sorbitol inférieure ou égale à 5 %, par rapport au poids total de matière sèche de la composition.

Cette fraction de fructose finale débarrassée en grande partie du sorbitol est d'abord concentrée à une concentration en sucres de l'ordre de 60 %, par rapport au poids total de la fraction (fraction humide), au moyen d'un évaporateur sous vide 21 fonctionnant à basse température, puis est recueillie dans la cuve 22.

Avant de pouvoir être commercialisé, ce sirop de fructose est soumis à un ensemble de traitements de finition, en vue de sa déminéralisation, sa désodorisation, sa décoloration et l'élimination de la patuline potentiellement présente, et pour améliorer sa stabilité dans le temps.

Dans un premier temps, le sirop de fructose est décoloré et déminéralisé par chromatographie sur un lit mélangé de résines, réalisé par mélange de deux résines : un échangeur cationique fortement acide et un échangeur anionique fortement basique.

A cet effet, deux colonnes de chromatographie 23a et 23b sont utilisées en série. Dès la saturation obtenue sur la première colonne 23a, celle-ci est régénérée. La totalité du flux passe alors sur la deuxième colonne 23b. Après régénération, la première colonne 23a est utilisée en deuxième position et le flux passe de nouveau sur deux colonnes en série.

En sortie de ce lit mélangé de résines, le taux de cendres conductimétriques de la composition n'est plus que de l'ordre de 0,2 %, voire bien inférieur.

Le sirop de fructose décoloré et déminéralisé est recueilli dans une cuve 24.

Dans un deuxième temps, le sirop de fructose est traité sur des charbons actifs, pour le débarrasser des odeurs d'amine, de la présence éventuelle de la patuline, de la coloration résiduelle. Le traitement est réalisé au niveau de la colonne 25. La température de traitement est de 60°C.

Un piège à charbon 26 est prévu en sortie de la colonne 25.

Le sirop de fructose ainsi traité est finalement concentré jusqu'à une concentration en sucres de l'ordre de 70 %, par rapport au poids total de la composition, au moyen d'un évaporateur sous vide 27 fonctionnant à basse température.

Le sirop de fructose final, de concentration en sucres de l'ordre de 70 %, par rapport au poids total de la composition, est stocké dans les réservoirs 28 en attendant d'être conditionné en tant que produit final, prêt à être consommé tel quel, et/ou en attendant d'être envoyé vers d'autres industries agroalimentaires de transformation.

Le procédé selon l'invention décrit ci-dessus a permis en particulier, en mettant en oeuvre tout ou partie des étapes, d'obtenir des sirops de sucres à partir de jus de pommes (exemple 1), jus de pèches (exemple 2) d'un mélange de jus de melon et de pèches (exemple 3).

### Exemple 1 :

Le procédé de préparation selon l'invention d'un sirop de fructose selon l'invention a été réalisé à partir d'un jus de pommes préalablement concentré. Les caractéristiques physicochimiques du jus initial et du sirop obtenu sont données dans le tableau 1 suivant :

**Tableau 1**

| Caractéristique physicochimiques | Sirop de fructose |
|---|---|
| °Brix réfractométrique à 20°c | 70 |
| pH (+/- 1) à 30° Brix | 4,68 |
| Cendres conductimétriques en g%g | 0,002 |
| Conductivité µS/cm à 28°B | 4 |
| Couleur (en ICUMSA) à 50° Brix | <2 |
| Fructose (% sucres totaux) | 98,9% |
| Glucose (% sucres totaux) | 0,9% |
| Saccharose (% sucres totaux) | 0,2% |
| Sorbitol / sucres totaux en % | <4,5% |

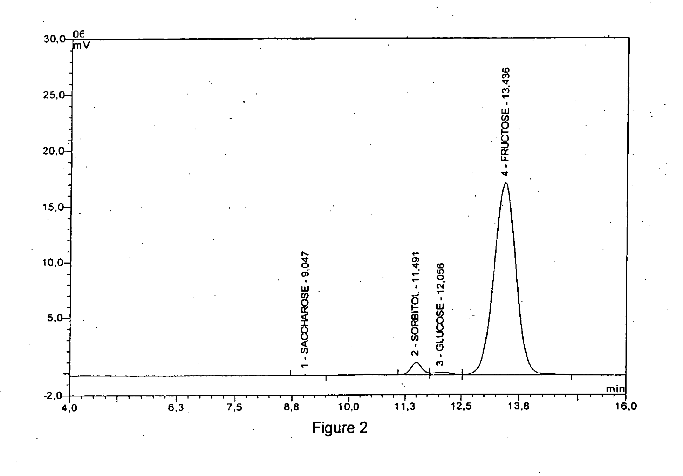

La figure 2 présente le spectre HPLC du sirop de sucre de pommes obtenu.

L'analyse de ce sirop a été réalisée dans les conditions opératoires suivantes :
colonne : BIORAD HPX87 K
éluant : Eau ultrapure
débit : 0,6 ml.min⁻¹
température : 65°C.

Le tableau 2 ci-après rapporte les résultats de dosage obtenu.

**Tableau 2**

| pic | rétention (min) | composé | taux de surface (%) | concentration (g.l⁻¹) |
|---|---|---|---|---|
| | | | | |
| 1 | 9,05 | saccharose | 0,23 | 0,018 |
| 2 | 11,49 | sorbitol | 3,33 | 0,254 |
| 3 | 12,06 | glucose | 0,93 | 0,072 |
| 4 | 13,44 | fructose | 95,51 | 7,448 |
| | | | | |
| Total : | | | 100,00 | 7,791 |

### Exemple 2 :

Le procédé de préparation selon l'invention d'un sirop de fructose selon l'invention a été réalisé à partir d'un jus de pêches. Les caractéristiques physicochimiques du jus initial et du sirop obtenu sont données dans le tableau 3 suivant.

**Tableau 3**

| Caractéristique physicochimiques | Sirop de fructose |
|---|---|
| °Brix réfractométrique à 20°c | 70- |
| pH (+/- 1) à 30° Brix | 4,5 +/- 0,5 |
| Couleur (en ICUMSA) à 50° Brix | <45 |
| Fructose (% sucres totaux) | 98,8 |
| Glucose (% sucres totaux) | 1,2 |
| Saccharose (% sucres totaux) | 0,01 |
| Sorbitol / sucres totaux en % | < 4,5% |

### Exemple 3 :

Compte tenu des exemples 1 et 2, une composition de sirop de fructose présentant une teneur en fructose au moins égal à 95 % par rapport au poids total de matière sèche et pouvant être obtenue conformément à l'invention, peut être déterminée grâce à une méthode de calcul prenant en compte les différents paramètres de la chromatographie.

La matière première végétale de départ peut être issue d'une part des fruits contenant du sorbitol, d'autre part des fruits ne contenant pas de sorbitol qui sont mélangés avec les précédents.

Le tableau 4 ci-après présente une liste non exhaustive des fruits contenant systématiquement du sorbitol, mais également des fruits pouvant contenir du sorbitol, selon leur origine et/ou leur variété. Les concentrations en sucres totaux (valeur haute et valeur basse) dans ces fruits ainsi que les concentrations en sorbitol (valeur haute et valeur basse) sont également répertoriées :

**Tableau 4**

| | Sucres totaux g%g de fruits | Sorbitol g%g de fruit | Pourcentage moyen de sorbitol/sucres totaux+sorbitol |
|---|---|---|---|
| Prune (2) | 8-18,6 | 7,4-8,6 | 37,4 % |
| Poire (1) | 7,9-13,3 | 1,21-2,8 | 17,6 % |
| Cerise (2) | 14,4 | 2,9 | 16.9 % |
| Prune (1) | 5,2-13,2 | 2,0-0,6 | 16,0 % |
| Cerise (1) | 11,9-24,8 | 1,4 | 7,7 % |
| Nectarine | 7,3-8,6 | 0,6-0,7 | 7,6 % |
| Abricot | 3,1-12,4 | 0,12-1,2 | 4,9 % |
| Pomme (1) | 9-14 | 0,2-1,0 | 4,7 % |
| Pèche | 8,9 | 0,4 | 4,5 % |

Les valeurs mentionnées dans le tableau 5 proviennent d'analyses réalisées par les inventeurs par CLHP, et des publications suivantes :
(1) Free sugars sorbitol fruits compilation from literature (1981). R.E. Wrolstad, R.S. Shallenberger, JAOAC, 64, 91-103.
(2) Compositional Characterization of prune juice (1992). H. van Gorsel and coll., J. Agric. Food Chem., 40, 784-789.

D'autres jus issus de matière première végétale ne contenant pas de sorbitol peuvent être utilisés en mélange avec les jus ou concentrés de fruits décrits précédemment, par exemple au moins un jus choisi parmi les jus d'agrumes, les jus de mélasse d'agrume, les jus de kiwi et les jus de melon, cette liste n'étant pas exhaustive.

A partir d'analyses réalisées sur des jus de fruit du commerce la composition de sirops de fructose pouvant être obtenus selon l'invention a été simulée. Trois exemples sont donnés dans les tableaux 5 à 7 ci-dessous : jus orange/pruneau (tableau 5), jus kiwi/poire (tableau 6) et jus nectarine/raisin (tableau 7). Les proportions choisies correspondent ici à 50 % de jus A pour 50 % de jus B, mais peuvent varier de 1 à 99 % et les combinaisons décrites ne sont pas restrictives.

**Tableau 5**

| Caractéristique physicochimiques | Sirop de fructose |
|---|---|
| °Brix réfractométrique à 20°c | 70+/-2 |
| pH (+/- 1) à 30° Brix | 4,5 +/- 0,5 |
| Cendres conductimétriques en g%g | <0,1 g%g |
| Couleur (en ICUMSA) à 50° Brix | <45 |
| Fructose (% sucres totaux) | 98,3 |
| Glucose (% sucres totaux) | 1,7 |
| Saccharose (% sucres totaux) | 0 |
| Sorbitol / sucres totaux | < 4,5% |

**Tableau 6**

| Caractéristique physicochimiques | Sirop de fructose |
|---|---|
| °Brix réfractométrique à 20°c | 70+/-2 |
| pH (+/- 1) à 30° Brix | 4,5 +/- 0,5 |
| Cendres conductimétriques en g%g | <0,1 g%g |
| Couleur (en ICUMSA) à 50° Brix | <45 |
| Fructose (% sucres totaux) | 97,9 |
| Glucose (% sucres totaux) | 2,1 |
| Saccharose (% sucres totaux) | 0 |
| Sorbitol / sucres totaux | <4.5% |

**Tableau 7**

| Caractéristique physicochimiques | Sirop de fructose |
|---|---|
| °Brix réfractométrique à 20°c | 70+/-2 |
| pH (+/- 1) à 30° Brix | 4,5 +/- 0,5 |
| Cendres conductimétriques en g%g | <0,1 g%g |
| Couleur (en ICUMSA) à 50° Brix | <45 |
| Fructose (% sucres totaux) | 98,3 |
| Glucose (% sucres totaux) | 1,7 |
| Saccharose (% sucres totaux) | 0 |
| Sorbitol / sucres totaux (%) | < 4,5% |

## Revendications

1. - Procédé de préparation d'un sirop de sucres à haute teneur en fructose, dans lequel :
- à partir d'au moins une matière première végétale de départ, on prépare un jus sucré clarifié et déminéralisé,
- on traite ledit jus sucré clarifié et déminéralisé de façon à hydrolyser le saccharose en fructose et en glucose ; on obtient une composition de sucres simples comprenant une fraction de fructose, dite première fraction de fructose, et une fraction de glucose,
- on sépare la fraction de glucose de la première fraction de fructose, et on réalise une isomérisation en fructose du glucose contenu dans cette fraction de glucose, pour former une nouvelle fraction de fructose, dite seconde fraction de fructose,
- on combine les première et seconde fractions de fructose, et on les concentre en un sirop de sucres riche en fructose, ledit procédé étant **caractérisé en ce que** :
1) au moins une matière première végétale de départ est issue d'au moins un fruit contenant naturellement du sorbitol,
2) il comprend une étape d'élimination au moins partielle du sorbitol.

2. - Procédé selon la revendication 1, **caractérisé en ce que** ladite matière végétale est issue d'au moins un fruit choisi parmi : les pommes, les poires, les prunes, les pruneaux, les pêches, les nectarines, les abricots, les raisins.

3. - Procédé selon l'une des revendications 1 ou 2, **caractérisé en ce que**, à partir de ladite matière végétale, on extrait un premier jus que l'on traite pour obtenir un jus sucré clarifié et déminéralisé de couleur inférieure à 45 ICUMSA, et présentant un taux de cendres conductimétriques inférieur à 0,4 %.

4. - Procédé selon la revendication 3, **caractérisé en ce que** pour obtenir ledit jus sucré clarifié et déminéralisé, on soumet le premier jus aux étapes de traitement suivantes :
- une centrifugation de l'ordre de 5 000-14 000 g,
- une ultrafiltration sur une membrane poreuse de seuil de coupure compris entre 1 kDa et 50 kDa,
- une électrodialyse ; on choisit des paramètres de travail adaptés pour permettre une élimination au moins partielle des charges ioniques dudit premier jus ;
- une chromatographie sur une résine échangeuse d'anions et une chromatographie sur une résine échangeuse de cations.

5. - Procédé selon la revendication 4, **caractérisé en ce qu'**on réalise l'électrodialyse avec des paramètres de travail choisis pour permettre l'obtention d'une composition liquide de conductivité à 50°C inférieure à 800 µS.cm⁻¹.

6. - Procédé selon l'une des revendications 4 et 5, **caractérisé en ce qu'**on réalise lesdites chromatographies sur résines échangeuses d'ions avec une résine cationique forte et une résine anionique faible avec des paramètres de travail adaptés pour permettre l'obtention d'un jus sucré décoloré et déminéralisé ayant un taux de cendres conductimétriques inférieur à 0,4 %.

7. - Procédé selon l'une des revendications 1 à 6, **caractérisé en ce qu'**on utilise une enzyme à activité β-D-fnictofilranosidase sous forme immobilisée pour réaliser l'hydrolyse du saccharose contenu dans le jus sucré décoloré et déminéralisé.

8. - Procédé selon l'une des revendications 1 à 7, **caractérisé en ce que**, pour convertir le glucose en fructose, on utilise une enzyme à activité glucose-isomérase sous une forme immobilisée.

9. - Procédé selon l'une des revendications 1 à 8, **caractérisé en ce qu'**à partir de la composition de sucres simples on réalise les étapes de purification et d'isomérisation suivantes :
- on soumet ladite composition de sucres simples à une chromatographie d'élution sur une colonne de résine cationique Ca²⁺ adaptée à une séparation glucose-fructose ; on obtient ladite première fraction de fructose et ladite fraction de glucose,
- on soumet ensuite la fraction de glucose à une isomérisation du glucose en fructose, puis on réalise une chromatographie d'élution sur une colonne de résine cationique Ca²⁺ adaptée à une séparation glucose-fructose ; on récupère ladite seconde fraction de fructose,
- on combine lesdites première et seconde fractions de fructose pour former une nouvelle fraction de fructose que l'on soumet à une chromatographie d'élution sur une colonne de résine cationique Ca²⁺ adaptée à une séparation fructose-sorbitol ; on choisit des paramètres de travail adaptés pour permettre d'obtenir une fraction de fructose finale de teneur en sorbitol inférieure ou égale à 5 %, par rapport au 7 poids total de matière sèche de la fraction.

10. - Procédé selon la revendication 9, **caractérisé en ce qu'**on soumet la fraction de fructose finale à :
- une déminéralisation par chromatographie sur résines échangeuses d'ions,
- un traitement sur des charbons actifs,
- une étape de concentration.

11. - Procédé selon la revendication 10, **caractérisé en ce qu'**on réalise ladite déminéralisation avec des paramètres de travail adaptés pour permettre l'obtention d'une composition au taux de cendres conductimétriques inférieur à 0,2 %.

12. - Procédé selon l'une des revendications 1 à 11, **caractérisé en ce que**, pour éviter les risques de contaminations, on concentre jusqu'à obtenir une concentration en sucres au moins de l'ordre de 60 %, par rapport au poids total de la composition, au moins une composition de sucres choisie parmi : ledit jus sucré décoloré et déminéralisé, ladite composition de sucres simples, une desdites fractions de fructose, ladite fraction de glucose.

13. - Installation pour la préparation d'un sirop de sucres à teneur élevée en fructose comprenant :
• des moyens d'extraction adaptés pour permettre d'extraire un premier jus, à partir d'au moins une matière première végétale de départ issue d'au moins un fruit contenant naturellement du sorbitol ;
• un équipement de raffinage pour préparer, à partir dudit premier jus, un jus sucré décoloré et déminéralisé de couleur inférieure à 45 ICUMSA, et ayant un taux de cendres conductimétriques inférieur à 0,4 %,
• un réacteur (13) comprenant une enzyme à activité β-D-fructofuranosidase,
• des moyens (15) permettant de réaliser une séparation d'une fraction de fructose et d'une fraction de glucose à partir ladite composition de sucres simples,
• un réacteur (19) comprenant une enzyme à activité glucose-isomérase,
• des moyens permettant une élimination du sorbitol.

14. - Installation selon la revendication 13, **caractérisée en ce qu'**elle comprend une colonne (15) de chromatographie d'élution renfermant une résine cationique Ca²⁺, et dotée de deux vannes de sortie à fonctionnement alterné, adaptée pour pouvoir réaliser sur commande soit une séparation glucose-fructose soit une séparation fructose-sorbitol.

15. - Installation selon l'une des revendications 13 ou 14, **caractérisée en ce qu'**elle comprend un équipement adapté pour réaliser un traitement de finition d'une composition de fructose consistant en une déminéralisation, une désodorisation, une décoloration, une élimination de la patuline potentiellement présente, et une concentration ; ledit équipement comprend :
- une colonne de chromatographie (23a ; 23b) renfermant un lit mélangé de deux résines : un échangeur cationique fortement acide et un échangeur anionique fortement basique,
- une colonne (25) de charbons actifs, couplée en sortie à un dispositif de filtration,
- un évaporateur fonctionnant sous vide et à basse température.

## Claims

1. A method for producing a sugar syrup having a high fructose content, **characterized in that**:
- a clarified and demineralised sweet juice is prepared from at least one initial plant raw material,
- said clarified and demineralised sweet juice is processed in order to hydrolyse the sucrose into fructose and glucose; a composition of simple sugars is obtained containing a fructose fraction, referred to as the first fructose fraction, and a glucose fraction,
- the glucose fraction is separated from the first fructose fraction and the glucose contained in this glucose fraction is isomerised into fructose to form a new fructose fraction referred to as the second fructose fraction,
- the first and second fructose fractions are combined and are concentrated into a high-fructose sugar syrup,
**characterized in that** in said method:
1) at least one initial plant raw material is obtained from at least one fruit naturally containing sorbitol,
2) an at least partial sorbitol elimination stage is included.

2. A method according to claim 1, **characterized in that** said plant material is obtained from at least one fruit selected from: apples, pears, plums, prunes, peaches, nectarines, apricots, grapes.

3. A method according to one of claims 1 or 2, **characterized in that** a first juice is extracted from said plant material and processed to obtain a clarified and demineralised sweet juice having a colour lower than 45 ICUMSA and having a conductometric ash content lower than 0.4%.

4. A method according to claim 3, **characterized in that** the first juice is put through the following processing stages in order to obtain said clarified and demineralised sweet juice:
- centrifugation in the order of 5000 to 14000 g,
- ultrafiltration through a porous membrane having a cut-off of between 1 kDa and 50 kDa,
- electrodialysis; suitable operating parameters are selected so as to allow at least partial elimination of the ionic charges from said first juice;
- chromatography on an anion-exchange resin and chromatography on a cation-exchange resin.

5. A method according to claim 4, **characterized in that** electrodialysis is performed with operating parameters chosen so as to obtain a liquid composition having a conductivity at 50°C of less than 800 µ.S.cm⁻¹.

6. A method according to one of claims 4 and 5, **characterized in that** said chromatography stages are performed on ion-exchange resins with a strong cationic resin and a weak anionic resin with suitable operating parameters to obtain a decolourised and demineralised sweet juice having a conductometric ash content lower than 0.4%.

7. A method according to one of claims 1 to 6, **characterized in that** an enzyme having β-D-fructofuranosidase activity in immobilised form is used to perform hydrolysis of the sucrose contained in the decolourised and demineralised sweet juice.

8. A method according to one of claims 1 to 7, **characterized in that** an enzyme having glucose isomerase activity in immobilised form is used to convert the glucose into fructose.

9. A method according to one of claims 1 to 8, **characterized in that** the composition of simple sugars is subjected to the following purification and isomerisation stages:
- said composition of simple sugars is subjected to elution chromatography on a Ca²⁺ cationic resin column suitable for glucose-fructose separation; said first fructose fraction and said glucose fraction are obtained,
- the glucose fraction is then subjected to isomerisation of glucose into fructose, then elution chromatography is performed on a Ca²⁺ cationic resin column suitable for glucose-fructose separation; said second fructose fraction is recovered,
- said first and second fructose fractions are combined to form a new fructose fraction which is subjected to elution chromatography on a Ca²⁺ cationic resin column suitable for fructose-sorbitol separation; suitable operating parameters are chosen so as to obtain a final fructose fraction having a sorbitol content of less than or equal to 5%, relative to the total weight of dry matter in the fraction.

10. A method according to claim 9, **characterized in that** the final fructose fraction is subjected to:
- demineralisation by chromatography on ion-exchange resins,
- activated carbon treatment,
- a concentration stage.

11. A method according to claim 10, **characterized in that** said demineralisation is performed with suitable operating parameters so as to obtain a composition having a conductometric ash content lower than 0.2%.

12. A method according to one of claims 1 to 11, **characterized in that** to avoid the risk of contamination concentration is performed until a sugar concentration is obtained in the region of at least 60% relative to the total weight of the composition on at least one sugar composition selected from: said decolourised and demineralised sweet juice, said composition of simple sugars, one of said fructose fractions, said glucose fraction.

13. An installation for producing a high-fructose sugar syrup comprising:
• suitable means of extraction for extracting a first juice from at least one initial plant raw material obtained from at least one fruit naturally containing sorbitol;
• refining equipment for producing from said first juice a decolourised and demineralised sweet juice having a colour lower than 45 ICUMSA and having a conductometric ash content lower than 0.4%,
• a reactor (13) comprising an enzyme having β-D-fructofuranosidase activity,
• means (15) of separating a fructose fraction and a glucose fraction starting from said composition of simple sugars,
• a reactor (19) comprising an enzyme having glucose isomerase activity,
• means of eliminating sorbitol.

14. An installation according to claim 13, **characterized in that** it includes an elution chromatography column (15) having a Ca²⁺ cationic resin and having two alternately operating outlet valves, suitable for performing either glucose-fructose separation or fructose-sorbitol separation as required.

15. An installation according to one of claim 14, **characterized in that** it includes suitable equipment for performing a final treatment of a fructose composition comprising demineralisation, deodorisation, decolourisation, elimination of any patulin that may be present, and concentration; said equipment includes:
- a chromatography column (23a; 23b) having a mixed bed of two resins: a highly acidic cation-exchange resin and a highly alkaline anion-exchange resin,
- an activated carbon column (25), the outlet from which is connected to a filtration device,
- a low-temperature vacuum evaporator.

## Patentansprüche

1. Verfahren zur Herstellung eines Fruchtzuckersirups mit hohem Fructosegehalt, bei dem:
- ausgehend von mindestens einem pflanzlichen Ausgangsrohstoff ein geklärter und entmineralisierter Zuckersaft zubereitet wird,
- der besagte geklärte und entmineralisierte Zuckersaft so behandelt wird, daß die Saccharose in Fructose und in Glucose hydrolysiert wird; es eine Zusammensetzung aus Einfachzucker erzielt wird, die eine Fructosefraktion, die sogenannte erste Fructosefraktion, und eine Glucosefraktion umfaßt,
- die Glucosefraktion von der ersten Fructosefraktion getrennt wird, und es eine Isomerisierung der in dieser Glucosefraktion enthaltenen Glucose in Fructose realisiert wird, um eine neue Fructosefraktion, die sogenannte zweite Fructosefraktion, zu bilden,
- die erste und die zweite Fructosefraktion kombiniert werden, und sie werden zu einem Zuckersirup mit hohem Fructoseanteil konzentriert,
wobei dieses Verfahren **dadurch gekennzeichnet ist, daß**:
1) zumindest ein pflanzlicher Ausgangsrohstoff aus zumindest einem Obst hervorgegangen ist, das von Natur aus Sorbit enthält,
2) es einen Schritt mit zumindest teilweiser Eliminierung des Sorbits umfaßt.

2. Verfahren nach Anspruch 1, **dadurch gekennzeichnet, daß** der besagte pflanzliche Stoff aus mindestens einem Obst hervorgegangen ist, das unter den folgenden gewählt wurde: Äpfel, Birnen, Pflaumen, Backpflaumen, Pfirsiche, Nektarinen, Aprikosen, Trauben.

3. Verfahren nach einem beliebigen der vorstehenden Ansprüche 1 oder 2, **dadurch gekennzeichnet, daß** ausgehend vom besagten pflanzlichen Stoff ein Rohsaft extrahiert wird, der verarbeitet wird, um einen geklärten und entmineralisierten Zuckersaft mit einer Farbe unter 45 ICUMSA zu erzielen, und der einen Anteil an Aschen für Leitfähigkeitsmessung von unter 0,4% aufweist.

4. Verfahren nach Anspruch 3, **dadurch gekennzeichnet, daß** der Rohsaft folgenden Behandlungsschritten unterzogen wird, um den besagten geklärten und entmineralisierten Zuckersaft zu erzielen:
- ein Zentrifugieren in Höhe von 5.000 - 14.000 g,
- eine Ultrafiltration auf einer porösen Membran mit Ausschlußgrenze zwischen 1 kDa und 50 kDa,
- eine Elektrodialyse; es werden Arbeitsparameter gewählt, die geeignet sind, eine zumindest teilweise Eliminierung der Ionenladungen des besagten Rohsafts zu ermöglichen,
- eine Chromatographie auf einem Anionenaustauscher und eine Chromatographie auf einem Kationenaustauscher.

5. Verfahren nach Anspruch 4, **dadurch gekennzeichnet, daß** die Elektrodialyse mit Arbeitsparametern durchgeführt wird, die so gewählt werden, daß eine flüssige Zusammensetzung mit Leitfähigkeit bei 50°C von unter 800 µS.cm⁻¹ erzielt werden kann.

6. Verfahren nach einem beliebigen der vorstehenden Ansprüche 4 und 5, **dadurch gekennzeichnet, daß** die besagten Chromatographien auf Ionenaustauschern mit einem starken Kationenharz und einem schwachen Anionenharz mit geeigneten Arbeitsparametern durchgeführt werden, die geeignet sind, einen entfärbten und entmineralisierten Zuckersaft mit einem Anteil an Aschen für Leitfähigkeitsmessung von unter 0,4% zu erzielen.

7. Verfahren nach einem beliebigen der vorstehenden Ansprüche 1 bis 6, **dadurch gekennzeichnet, daß** ein Enzym mit Beta-D-Fructofuranosidase-Aktivität in immobilisierter Form für die Hydrolyse der Saccharose eingesetzt wird, die im entfärbten und entmineralisierten Zuckersaft enthalten ist.

8. Verfahren nach einem beliebigen der vorstehenden Ansprüche 1 bis 7, **dadurch gekennzeichnet, daß** für die Umwandlung von Glucose in Fructose ein Enzym mit Glucose-Isomerase-Aktivität in immobilisierter Form eingesetzt wird.

9. Verfahren nach einem beliebigen der vorstehenden Ansprüche 1 bis 8, **dadurch gekennzeichnet, daß** ausgehend von der Einfachzucker-Zusammensetzung die nachstehenden Schritte Purifizierung und Isomerisierung durchgeführt werden:
- die besagte Einfachzucker-Zusammensetzung wird einer Eluierungs-Chromatographie auf einer Kationenharzsäule Ca²⁺ unterzogen, die für eine Trennung von Glucose und Fructose geeignet ist; es wird die besagte erste Fructosefraktion und die besagte Glucosefraktion erzielt,
- anschließend wird die Glucosefraktion einer Isomerisierung der Glucose in Fructose unterzogen, und dann wird eine Eluierungs-Chromatographie auf einer Kationenharzsäule Ca²⁺ durchgeführt, die für eine Trennung von Glucose und Fructose geeignet ist; die besagte zweite Fructosefraktion wird gewonnen,
- die besagten erste und zweite Fructosefraktionen werden kombiniert, um eine neue Fructosefraktion zu bilden, die einer Eluierungs-Chromatographie auf einer Kationenharzsäule Ca²⁺ unterzogen wird, die für eine Trennung von Glucose und Sorbit geeignet ist; es werden geeignete Arbeitsparameter gewählt, damit eine finale Fructosefraktion mit einem Sorbitgehalt unter oder gleich 5% erzielt werden kann im Verhältnis zum 7 Gesamtgewicht Trockenmasse der Fraktion.

10. Verfahren nach Anspruch 9, **dadurch gekennzeichnet, daß** die finale Fructosefraktion folgende Schritte durchläuft:
- eine Entmineralisierung durch Chromatographie auf Ionenaustauschern,
- eine Behandlung auf Aktivkohle,
- ein Schritt mit Konzentration.

11. Verfahren nach Anspruch 10, **dadurch gekennzeichnet, daß** die besagte Entmineralisierung mit geeigneten Arbeitsparametern durchgeführt wird, damit eine Zusammensetzung mit einem Anteil an Aschen für Leitfähigkeitsmessung von unter 0,2% erzielt werden kann.

12. Verfahren nach einem beliebigen der vorstehenden Ansprüche 1 bis 11, **dadurch gekennzeichnet, daß** zur Vermeidung von Kontaminationsrisiken so lange konzentriert wird, bis eine Zuckerkonzentration von mindestens um die 60% erzielt wird, und im Verhältnis zum Gesamtgewicht der Zusammensetzung zumindest eine Zusammensetzung von Zuckern, die gewählt wird unter: dem besagten entfärbten und entmineralisierten Zuckersaft, der besagten Einfachzucker-Zusammensetzung, einer der besagten Fructosefraktionen, der besagten Glucosefraktion.

13. Anlage zur Herstellung eines Zuckersirups mit hohem Fructosegehalt, die folgende Teile umfaßt:
. Extraktionsmittel, die geeignet sind, einen Rohsaft zu extrahieren, und dies ausgehend von zumindest einem pflanzlichen Ausgangsrohstoff, der aus zumindest einem Obst hervorgegangen ist, das von Natur her Sorbit enthält;
. Eine Raffinationsausrüstung zum Zubereiten ausgehend vom besagten Rohsaft eines entfärbten und entmineralisierten Zuckersafts mit einer Farbe von unter 45 ICUMSA und mit einem Anteil an Aschen für Leitfähigkeitsmessung von unter 0,4%,
. Ein Reaktionsgefäß (13) mit einem Enzym mit Beta-D-Fructofuranosidase-Aktivität,
. Mittel (15) für die Durchführung einer Trennung einer Fructosefraktion von einer Glucosefraktion ausgehend von besagter Einfachzucker-Zusammensetzung,
. Ein Reaktionsgefäß (19) mit einem Enzym mit Glucose-Isomerase-Aktivität,
. Mittel für die Eliminierung des Sorbits.

14. Anlage nach Anspruch 13, **dadurch gekennzeichnet, daß** sie eine Säule (15) für die Eluierungs-Chromatographie umfaßt, die ein Kationenharz Ca²⁺ umschließt, und die mit zwei Ausgangsschiebern mit abwechselndem Betrieb versehen und geeignet ist, auf Steuerung entweder eine Glucose-Fructose-Trennung oder eine Fructose-Sorbit-Trennung durchzuführen.

15. Anlage nach einem beliebigen der vorstehenden Ansprüche 13 oder 14, **dadurch gekennzeichnet, daß** sie eine Ausrüstung umfaßt, die geeignet ist, eine Endbearbeitung einer Fructosezusammensetzung durchzuführen, die aus einer Entmineralisierung, einer Desodorisierung, einer Entfärbung, einer Eliminierung des potentiell vorhandenen Patulins und einer Konzentration besteht; die besagte Ausrüstung umfaßt folgende Teile:
- eine Chromatographie-Säule (23a; 23b), die ein Bett mit einem Gemisch aus zwei Harzen umschließt: ein stark saurer Kationenaustauscher und ein stark basischer Anionenaustauscher,
- eine Aktivkohle-Säule (25), die am Ausgang mit einer Filtrationsvorrichtung gekoppelt ist,
- einen Verdampfer, der im Vakuum und bei niedriger Temperatur funktioniert.
